# EUROPEAN PATENT SPECIFICATION

(11) **EP 3 796 887 B1**
(45) Date of publication and mention of the grant of the patent: **19.06.2024**
(21) Application number: 19723313.3
(22) Date of filing: 10.05.2019
(51) Int. Cl.: A61K 8/37, A61K 8/39, A61K 8/46, A61K 8/42, A61Q 5/02, A61K 8/06, A61K 9/107, A61K 31/355, A61K 47/10, A61K 47/18, A61K 47/20

(54) **A PEDICULICIDAL SHAMPOO**
ANTILÄUSESHAMPOO
SHAMPOO PEDICULICIDE

(30) Priority: 22.05.2018 GB 201808398
(43) Date of publication of application: 31.03.2021
(73) Proprietor: Thornton & Ross Limited, Huddersfield HD7 5QH (GB)
(72) Inventor: COOPER, Nigel, Yorkshire HD7 5QH (GB); BRUNTON, Elizabeth Rachel, Cambridge CB259AU (GB)
(74) Representative: Meissner Bolte UK
(86) International application number: PCT/EP2019/025144
(87) International publication number: WO 2019/223901

(56) References cited:
- EP-A2- 0 968 706
- WO-A2-2008/038140
- GB-A- 2 550 558

## Description

The present invention relates to a shampoo for use in the treatment of pediculosis, in particular pediculosis capitis, according to the appended claims.

Many effective pediculicidal compositions are known for the treatment of head louse infestations. Traditionally, these have comprised conventional insecticides such as permethrin. However, many of these substances have an unpleasant odour and may cause allergic reactions. More recently non-toxic products have been found that are clinically effective. In particular, some silicone polymers, which are widely used in personal care products such as in shampoo and hair conditioners, have been found to be highly effective in eradicating both head lice and their ova when used in certain formulations, for example WO2001/019190 and WO2010/130983. However, these compositions have to be washed out of the hair after use. It would therefore be more convenient if a pediculicidal composition were to be formulated as a shampoo so that treatment could be combined with washing of the hair in a single operation.

Shampoos are generally made by combining a surfactant, most often sodium lauryl sulphate or sodium laureth sulphate, with a co-surfactant that acts as a foam booster so that once the hair is wetted with water during use a lather can be formed. Shampoos in liquid form usually contain a large proportion of water, which is used as the bulking agent because it is inert and miscible with other shampoo ingredients.

It will be appreciated, however, that conventional siloxane-based pediculicides cannot readily be incorporated into a shampoo without losing their efficacy as they act by coating the lice and ova with a siloxane film and must therefore be used in a non-aqueous environment.

In addition to the foregoing, even after treatment to kill head lice, the removal of ova and nits from the hair can be difficult as they cling tenaciously to individual hairs, usually within 10 mm of the scalp. This is because they are glued to the hair shaft by a hydrophobic glue that the louse secretes on to the hair shaft. The louse spreads the glue over the hair shaft by movement of her body and forms a mould into which she then lays the ovum, which is also substantially coated in the glue. The glue solidifies on exposure to oxygen in the atmosphere thereby firmly binding the ovum to the hair shaft. Simply shampooing using a conventional shampoo is not, therefore, sufficient to remove nits and ova from the hair and techniques conventionally used to remove lice ova and nits from the hair after treatment with pediculicidal compositions are the vigorous brushing of the hair or combing with a fine tooth nit comb. These techniques are inefficient and rarely succeed in removing a substantial quantity of the ova and nits because the louse glue is so effective. They are also painful and much more likely to damage the hair than remove the nits.

Previously, the applicant has found that an aqueous gel containing in excess of 5% by weight and preferably an optimum of 20% of a cosmetically-acceptable oil being any or a mixture of isopropyl myristate, isopropyl palmitate and isononyl isononanoate, a thickener and water is capable of loosening nits. This is described in WO2017/198982 wherein the gel is applied to the hair and left in place for a few minutes so that it commences work to loosen louse ova and nits before being washed out using a proprietary shampoo. It was thought that dilution of the aforesaid oils in a thickened aqueous gel enabled the oil to coat and to penetrate the louse glue more effectively, thereby degrading the glue and enabling a the louse ovum or nit to slide off the hair shaft when combed. As it was also known that aliphatic esters such as the aforesaid oils have an ectoparasiticidal effect the gel is also effective in killing live lice in addition to loosening their ova and nits. However, the gel also has the disadvantage of having to be washed out of the hair after use.

US4,147,800 describes a pediculicidal composition comprising an admixture of an aliphatic alcohol and an aliphatic ester. In particular a shampoo is described comprising 65% isopropyl alcohol, 10% isopropyl myristate, 20% trietheranolamine lauryl sulphate and water. However, this composition is not particularly effective in loosening nits or ova from the hair and it also contains a high quantity of alcohol, which is drying and not popular when used in shampoos and the like for use on young children on which most pediculicidal shampoos are used. Further compositions for use in the treatment of pediculosis are disclosed in GB 2 550 558A, in particular a louse ova and nit loosening composition.

An object of the present invention is therefore to provide a non-toxic, liquid shampoo for use in the treatment of pediculosis and, in particular, to a treatment shampoo that not only kills lice but that also enables louse ova and nits to be dislodged from the hair and washed away.

Surprisingly, it has been found that a non-aqueous, liquid shampoo formulation containing isononyl isononanoate as the sole active is capable of not only killing lice but also of enabling the removal of louse ova or nits from the hair when the shampoo is rinsed off after use.

As isononyl isononanoate has a very low solubility in water, the use of water as a bulking agent in a shampoo containing a high quantity of isononyl isononanoate is inappropriate. Isononyl isononanoate is soluble in ethanol but the use of alcohol in a shampoo primarily for use on children is also inappropriate and, in any event, would result in excessive drying of the hair. In addition, as isononyl isononanoate is an oil a means has to be found of successfully incorporating it in a shampoo as the more oil that is used to increase the pediculicidal efficacy of the shampoo, the less foam is likely to be produced thereby decreasing the effectiveness of the shampoo itself. Hence, finding an appropriate shampoo formulation that is safe to use on children and which contains a sufficient quantity of isononyl isononanoate for efficacy was not without difficulty.

According to the present invention there is provided a non-toxic, non-aqueous, liquid shampoo for use in the treatment of pediculosis, according to claim 1.

Tests have been conducted using varying quantities of the isononyl isononanoate as the active in the shampoo of the present invention. As indicated above, a balance has to be struck between the quantity of isononyl isononanoate used and the other shampoo ingredients. This is because isononyl isononanoate suppresses foaming of the shampoo but, as the active, a sufficient quantity is required for the shampoo to have an effective pediculicidal and ova/nit loosening effect. It has been found that shampoos containing at least 50 wt% inclusive produced the best results. At lower levels of isononyl isononanoate, the shampoo of the invention is still effective but not all of the lice may be killed and some ova and nits may be left in the hair. However, at levels between 50 wt% and 70 wt% inclusive the shampoo was found to have 100% efficacy while still enabling sufficient detergent foaming activity to occur to provide the cleaning effect required from the other constituents of the shampoo.

Shampoos formulated with quantities of isononyl isononanoate greater than 70 wt% while providing 100% pediculicidal and ova/nit loosening efficacy were found not to foam satisfactorily. Also, if the other constituents of the shampoo are increased to a level greater than 30 wt%, the shampoo was found to sting and be unacceptably irritating to the eyes.

One unexpected side effect of the use of isononyl isononanoate is that it is an emollient that mellows the eye-irritating effects of the other constituents. It will be appreciated that this is an important feature for shampoos that are to be used primarily on young children.

Preferably, therefore, the shampoo comprises between 50 wt% and 70 wt% inclusive isononyl isononanoate. Advantageously, the shampoo comprises 70 wt% isononyl isononanoate.

The detergent comprises a mixture of an anionic surfactant and a non-ionic emulsifying agent. Non-ionic emulsifying agents are better at emulsifying isononyl isononanoate but anionic surfactants have better shampooing qualities. The detergent therefore comprises a mixture of these two compounds. Advantageously, this is a mixture containing equal quantities of a non-ionic emulsifying agent and an anionic surfactant.

The emulsifying agent is preferably a polyoxyether of lauryl alcohol, such as laureth 4, which also acts as a surfactant and which enables a high quantity of the isononyl isononanoate to be incorporated into the shampoo. Advantageously, the shampoo may comprise up to 25 wt% of the emulsifying agent, which is preferably laureth 4.

The anionic surfactant is preferably a laureth sulphate, such as MIPA-laureth sulphate, which is a mild anionic surfactant and therefore suitable for use in a shampoo that will primarily be used on children. Advantageously, the shampoo may also comprise up to 25 wt% of the anionic surfactant, which is preferably laureth sulphate.

The detergent may also comprise a foaming agent. This is preferably a diethanolamide, advantageously cocamide diethanolamine. The shampoo preferably comprises up to 14 wt% of the foaming agent.

In some formulations up to 5 wt% inclusive of the shampoo may comprise small quantities of colouring agents, fragrance agents and other cosmetically-acceptable additives. None of these should be pharmacologically-active ingredients. Other formulations of the shampoo may contain none of these cosmetically-acceptable additives.

Advantageously, the composition has a pH in the range of 6 to 8 inclusive and is preferably pH neutral.

One specific example of a shampoo formulated in accordance with the invention is as follows.
50 wt% isononyl isononanoate
21 wt% MIPA-laureth sulphate
21 wt% laureth 4
8 wt% cocamide diethanolamine

Another, preferred example of shampoo formulated in accordance with the invention is as follows.
70 wt% isononyl isononanoate
13 wt% MIPA-laureth sulphate
13 wt% laureth 4
4 wt% cocamide diethanolamine

The compositions of the present invention may be prepared by any suitable means for producing emulsions. By way of example, the composition may be prepared by mixing the isononyl isononanoate with a previously prepared mixture of the other constituents of the shampoo.

In use, the shampoo is applied to wetted hair in the same way as a conventional cosmetic shampoo. However, as it is a liquid emulsion its container should be shaken or the shampoo itself vigorously stirred before application. After application it will lather and the lather should be massaged in to the hair and scalp so that all hair shafts are coated. The lather should then be left in place for around 10 minutes so that it commences work to kill lice and loosen louse ova and nits. The shampoos may be then rinsed off and the hair combed with a fine-toothed nit comb to remove dead lice, loosened ova and nits, care being taken to comb from hair roots to hair tips to ensure all ova and nits are acted on and satisfactorily removed from the hair shafts. Once the user is satisfied that all of the hair has been adequately combed, any remaining shampoo and louse debris can be rinsed off before the hair is dried.

To justify the present invention the following independent tests were conducted using the specific shampoo formulation given above, namely
50 wt% isononyl isononanoate
21 wt% MIPA-laureth sulphate
21 wt% laureth 4
8 wt% cocamide diethanolamine

### Test treatments and application

Head lice, namely *pediculus humanus capitis*, that had been ethically obtained from a family who requested assistance were counted into batches and provided with squares of open meshed nylon gauze, as a substrate upon which to stand. Each batch was allocated to a marked 55 millimetre plastic Petri dish. Each square of lice and gauze (4cm²) was then massaged with approximately 5 mls of the test product. This was performed on the dry gauze so that the products could be used neat and could be massaged in.

After the lice had been exposed to the test formulations for the appropriate time water was added to the dishes, they were stirred with the tip of a finger in an attempt to disperse the shampoo in the water and they were then rinsed using at least 500 millilitres of warm (34°C Celsius) tap water poured through and over the gauze squares. Finally they were blotted dry using medical wipe tissue, and incubated under normal maintenance conditions in clean plastic Petri dishes until the results were recorded.

Three replicates of each treatment were tested alongside three Control replicates, in which tap water was used in place of the shampoo under the same exposure conditions.

After the treatments had been washed off, the lice were observed for signs of recovery or degeneration. During these readings lice were categorised as 'Immobile', 'Moribund' and 'Alive'. 'Immobile' refers to a state of no movement, 'Moribund' describes slight uncoordinated movement and 'Alive' is used to describe lice with full movement.

### Results

Approximately 5 mL of shampoo and Control water was applied. The shampoo flowed readily and easily coated both lice and gauze with minimum additional disturbance, although the gauze was turned about to ensure complete coverage of all insects and the elimination of any air bubbles.

No recording of louse activity was made immediately after washing because some lice, even in the Control group, were still recovering from immersion in water.

Observations made at 2 hours, and 4 hours after wash-off showed a number of surviving lice in each of the treatment groups (see attached Table). There was no decline in viability between the 2 and 4 hours observations with a result of 89.4 % efficacy.

It was observed that those lice still alive at the 2 hour and 4 hour readings were first instar nymphs. When assessed the following morning, all of the lice were dead giving 100 % mortality.

### Conclusion

The shampoo preparation gave 100 % efficacy.

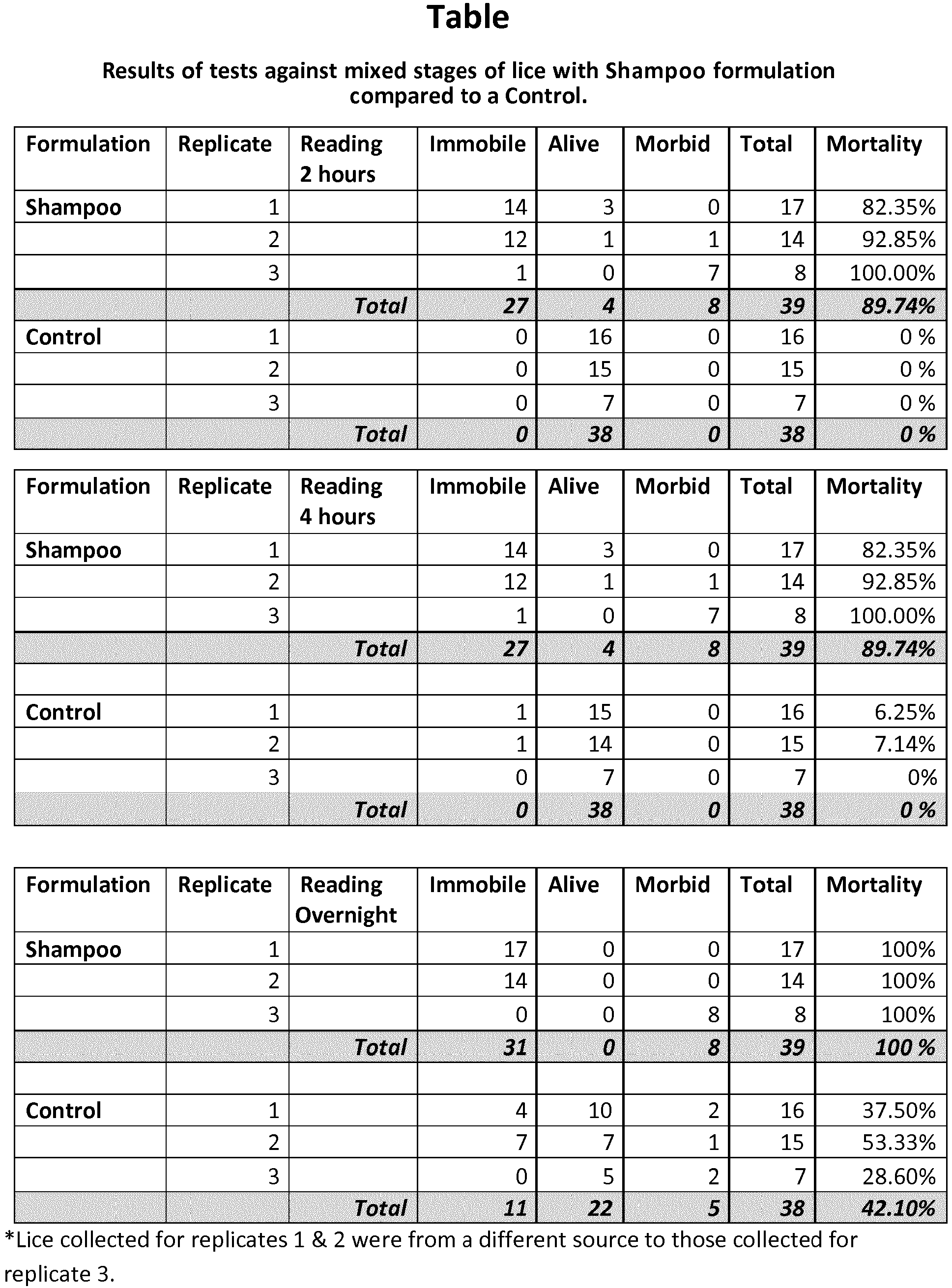

## Claims

1. A non-toxic, non-aqueous, liquid shampoo that does not contain alcohol, and that comprises an emulsion of at least 50 wt% isononyl isononanoate, up to 50 wt% inclusive of a detergent comprising a mixture of an anionic surfactant and a non-ionic emulsifying agent, and between 0 wt% to 5 wt% inclusive of one or more cosmetically-acceptable additives, wherein said shampoo is for use in the treatment of pediculosis.

2. A shampoo for use as claimed in Claim 1, comprising 70% isononyl isononanoate.

3. A shampoo for use as claimed in Claim 1 or 2, comprising at least 9 wt% of the emulsifying agent.

4. A shampoo for use as claimed in Claim 3, wherein the emulsifying agent is a polyoxyether of lauryl alcohol.

5. A shampoo for use as claimed in Claim 4, wherein the emulsifying agent is laureth 4.

6. A shampoo as for use as claimed in any of Claims 1 to 5, comprising at least 9 wt% of the anionic surfactant.

7. A shampoo for use as claimed in Claim 6, wherein the anionic surfactant is a laureth sulphate.

8. A shampoo for use as claimed in Claim 7, wherein the anionic surfactant is MIPA-laureth sulphate.

9. A shampoo for use as claimed in any of Claims 1 to 8, wherein the detergent additionally comprises a foaming agent.

10. A shampoo for use as claimed in Claim 9, comprising up to 8 wt% of the foaming agent.

11. A shampoo for use as claimed in Claim 9 or Claim 10, wherein the foaming agent is a diethanolamide.

12. A shampoo for use as claimed in Claim 11, wherein the foaming agent is cocamide diethanolamine.

13. A shampoo for use as claimed in any of Claims 1 to 12, comprised of 70 wt% isononyl isononanoate and a detergent comprised of 13wt% MIPA-laureth sulphate, 13 wt% laureth 4, and 4 wt% cocamide diethanolamine.

14. A shampoo for use as claimed in any of Claims 1 to 11, comprised of 50 wt% isononyl isononanoate and a detergent comprised of 21 wt% MIPA-laureth sulphate, 21 wt% laureth 4, and 8 wt% cocamide diethanolamine.

15. A shampoo for use as claimed in any of Claims 1 to 15, which has a pH between 6 and 8 inclusive, preferably which is pH neutral.

## Patentansprüche

1. Nicht-toxisches, nicht-wässriges, flüssiges Shampoo, das keinen Alkohol enthält und das eine Emulsion aus mindestens 50 Gew.-% Isononylisononanoat, bis zu 50 Gew.-% einschließlich eines Detergens, das eine Mischung aus einem anionischen Tensid und einem nicht-ionischen Emulgiermittel umfasst, und zwischen 0 Gew.-% und 5 Gew.-% einschließlich eines oder mehrerer kosmetisch akzeptabler Additive umfasst, wobei das Shampoo zur Verwendung bei der Behandlung von Pedikulose bestimmt ist.

2. Shampoo zur Verwendung nach Anspruch 1, umfassend 70 % Isononylisononanoat.

3. Shampoo zur Verwendung nach Anspruch 1 oder 2, umfassend mindestens 9 Gew.-% des Emulgiermittels.

4. Shampoo zur Verwendung nach Anspruch 3, wobei das Emulgiermittel ein Polyoxyether von Laurylalkohol ist.

5. Shampoo zur Verwendung nach Anspruch 4, wobei das Emulgiermittel Laureth-4 ist.

6. Shampoo zur Verwendung nach einem der Ansprüche 1 bis 5, umfassend mindestens 9 Gew.-% des anionischen Tensids.

7. Shampoo zur Verwendung nach Anspruch 6, wobei das anionische Tensid ein Laurethsulfat ist.

8. Shampoo zur Verwendung nach Anspruch 7, wobei das anionische Tensid MIPA-Laurethsulfat ist.

9. Shampoo zur Verwendung nach einem der Ansprüche 1 bis 8, wobei das Detergens zusätzlich einen Schaumbildner umfasst.

10. Shampoo zur Verwendung nach Anspruch 9, umfassend bis zu 8 Gew.-% des Schaumbildners.

11. Shampoo zur Verwendung nach Anspruch 9 oder Anspruch 10, wobei der Schaumbildner ein Diethanolamid ist.

12. Shampoo zur Verwendung nach Anspruch 11, wobei der Schaumbildner Cocamiddiethanolamin ist.

13. Shampoo zur Verwendung nach einem der Ansprüche 1 bis 12, bestehend aus 70 Gew.-% Isononylisononanoat und einem Detergens bestehend aus 13 Gew.-% MIPA-Laurethsulfat, 13 Gew.-% Laureth-4 und 4 Gew.-% Cocamiddiethanolamin.

14. Shampoo zur Verwendung nach einem der Ansprüche 1 bis 11, bestehend aus 50 Gew.-% Isononylisononanoat und einem Detergens bestehend aus 21 Gew.-% MIPA-Laurethsulfat, 21 Gew.-% Laureth-4 und 8 Gew.-% Cocamiddiethanolamin.

15. Shampoo zur Verwendung nach einem der Ansprüche 1 bis 15, das einen pH-Wert zwischen 6 und 8 einschließlich aufweist, vorzugsweise das pH-neutral ist.

## Revendications

1. Shampoing liquide non toxique, non aqueux, qui ne contient pas d'alcool et qui comprend une émulsion d'au moins 50 % en poids d'isononanoate d'isononyle, jusqu'à 50 % en poids inclusivement d'un détergent comprenant un mélange d'un tensioactif anionique et d'un agent émulsifiant non ionique, et entre 0 % et 5 % en poids inclusivement d'un ou plusieurs additifs acceptables sur le plan cosmétique, dans lequel ledit shampoing est destiné au traitement de la pédiculose.

2. Shampoing destiné à être utilisé selon la revendication 1, comprenant 70 % d'isononanoate d'isononyle.

3. Shampoing destiné à être utilisé selon la revendication 1 ou 2, comprenant au moins 9 % en poids de l'agent émulsifiant.

4. Shampoing destiné à être utilisé selon la revendication 3, dans lequel l'agent émulsifiant est un polyoxyéther d'alcool laurique.

5. Shampoing destiné à être utilisé selon la revendication 4, dans lequel l'agent émulsifiant est le laureth-4.

6. Shampoing destiné à être utilisé selon l'une quelconque des revendications 1 à 5, comprenant au moins 9 % en poids du tensioactif anionique.

7. Shampoing destiné à être utilisé selon la revendication 6, dans lequel le tensioactif anionique est un laureth sulfate.

8. Shampoing destiné à être utilisé selon la revendication 7, dans lequel le tensioactif anionique est le MIPA-laureth sulfate.

9. Shampoing destiné à être utilisé selon l'une quelconque des revendications 1 à 8, dans lequel le détergent comprend en outre un agent moussant.

10. Shampoing destiné à être utilisé selon la revendication 9, comprenant jusqu'à 8 % en poids de l'agent moussant.

11. Shampoing destiné à être utilisé selon la revendication 9 ou la revendication 10, dans lequel l'agent moussant est un diéthanolamide.

12. Shampoing destiné à être utilisé selon la revendication 11, dans lequel l'agent moussant est la cocamide diéthanolamine.

13. Shampoing destiné à être utilisé selon l'une quelconque des revendications 1 à 12, constitué de 70 % en poids d'isononanoate d'isononyle et d'un détergent constitué de 13 % en poids de MIPA-laureth sulfate, de 13 % en poids de laureth-4 et de 4 % en poids de cocamide diéthanolamine.

14. Shampoing destiné à être utilisé selon l'une quelconque des revendications 1 à 11, constitué de 50 % en poids d'isononanoate d'isononyle et d'un détergent constitué de 21 % en poids de MIPA-laureth sulfate, de 21 % en poids de laureth-4 et de 8 % en poids de cocamide diéthanolamine.

15. Shampoing destiné à être utilisé selon l'une quelconque des revendications 1 à 15, dont le pH est compris entre 6 et 8 inclusivement, de préférence dont le pH est neutre.
